# EUROPEAN PATENT APPLICATION

(11) **EP 2 361 967 A1**
(43) Date of publication of application: **31.08.2011**
(21) Application number: 10305194.2
(22) Date of filing: 26.02.2010
(51) Int. Cl.: C12N 5/00

(54) **Gametes separation methods, compositions and uses thereof**

(71) Applicant: Assistance Publique - Hôpitaux de Paris, 75004 Paris (FR)
(72) Inventor: Vega, Manuel, 91000, EVRY (FR); Drittanti, Lila, 8000, BAHIA BLANCA (AR); Goossens, Michel, 94240, L'HAY-LES-ROSES (FR)
(74) Representative: Starck-Loudes, Anne-Caroline

(57) **Abstract**

The present invention relates to a method of separating gametes of a subject, which method comprises discriminating a first population of gametes containing an abnormal nucleic acid sequence involved in a genetic disease or in a multifactorial disorder in the offspring of the subject, from a second population of gametes which does not contain said abnormal nucleic acid sequence. The invention further relates to products and compositions which may be used in such a method.

SEQUENCE LISTING

<110>  Assistance Publique Hôpitaux de Paris
       Assistance Publique - Hôpitaux de Paris

<120>  GAMETES SEPARATION METHODS, COMPOSITIONS AND USES THEREOF

<130>  B1004

<160>  6

<170>  PatentIn version 3.3

<210>  1
<211>  1480
<212>  PRT
<213>  Homo sapiens


<220>
<221>  MISC_FEATURE
<223>  human CFTR protein sequence

<400>  1

<210> 2
<211> 30
<212> DNA
<213> artificial sequence

<220>
<223> Primer sequence_ normal allele_ Antisens sequence

<400> 2

gacttcactt ctaatgatga ttatgggaga                                        30


<210>  3
<211>  30
<212>  DNA
<213>  artificial sequence

<220>
<223>  Primer sequence_ normal allele_ Sens sequence

<400>  3
gtatctatat tcatcatagg aaacaccaca                                        30


<210>  4
<211>  30
<212>  DNA
<213>  artificial sequence

<220>
<223>  Primer sequence_ mutant allele_ Sens sequence

<400>  4
gtatctatat tcatcatagg aaacaccatt                                        30


<210>  5
<211>  11
<212>  PRT
<213>  artificial sequence

<220>
<223>  artificial sequence


<220>
<221>  MISC_FEATURE
<222>  (1)..(1)
<223>  Xaa= Thr-Dabcyl

<220>
<221>  MISC_FEATURE
<222>  (11)..(11)
<223>  Xaa= Arg-EDANS

<400>  5

Xaa Pro Leu Lys Ser Pro Pro Pro Ser Pro Xaa
1               5                   10


<210>  6
<211>  19
<212>  PRT
<213>  artificial sequence

<220>
<223>  artificial sequence


<220>
<221>  MISC_FEATURE
<222>  (1)..(1)
<223>  Xaa= Thr-Dabcyl

<220>
<221>  MISC_FEATURE
<222>  (12)..(12)

<223>  Xaa= glu-EDANS

<400>  6

Xaa Pro Leu Lys Ser Pro Pro Pro Ser Pro Arg Xaa Arg Arg Arg Arg
1               5                   10                  15

Arg Arg Arg

## Description

The present invention relates to the field of medicine and medical research. Inventors herein describe a novel approach for the prevention of diseases involving, for a given subject, the separation of gamete populations.

More specifically, the present invention relates to a method of separating gametes of a subject, which method comprises discriminating at least a first population of gametes containing an abnormal nucleic acid sequence involved in a genetic disease or in a multifactorial disorder in the offspring of the subject, from a second population of gametes which does not contain said abnormal nucleic acid sequence. The invention further relates to products and compositions which may be used in such a method.

### BACKGROUND

The available means to prevent in a subject, or help reducing the detrimental consequences of, the onset of a disease at least partly due to the presence, in said subject, of an abnormal nucleic acid sequence, are very few and insufficiently effective.

In particular, the unsatisfactory means for human parents to prevent the appearance or onset of a genetic disease in their progeny is, depending on circumstances, either to avoid procreation or, when possible, to decide terminating pregnancy.

The indications for assisted reproductive techniques have greatly expanded in the last decade and are expected to further increase in the future (Katz P, Nachtigall R, Showstack J. "The economic impact of the assisted reproductive technologies.", Nat Cell Biol. 2002; 4: s29 -s32.).

In medicine, the expression "preimplantation genetic diagnosis" (PGD or PIGD) (also known as "embryo screening") refers to procedures that are performed on early-stage embryos prior to implantation. Its main advantage is that it avoids selective pregnancy termination as the method makes it highly likely that the baby will be free of the disease under consideration.

The term "preimplantation genetic screening" (PGS) is used to denote procedures that do not look for a specific disease but use PGD techniques to identify embryos at risk, i.e., embryos having a deleterious anatomical, physiological, or genetic condition that could lead to disease.

Deleterious conditions, characteristics or traits are those that limit or prevent fertilization and those that are detrimental to the future human being, at the various stages of its development (embryo, foetus, infant, child, adult, etc.). Deleterious characteristics are in particular those that are involved in a disease or disorder, such as, for example, an incapacitating disease, or a disease leading to the premature death of the human being.

Semen and oocyte assessment methods are used extensively in the context of medically assisted procreation and in particular in investigation of human clinical infertility.

During the last decade, laboratories carried out methods for evaluating the sperm and oocyte quality, providing information about the morphology, functional integrity and viability of male and female gametes, in particular the acrosomal integrity and motility of spermatozoids.

DNA damage in spermatozoids may be detected by using Raman spectroscopy. It is however not resolutive enough to detect specific traits (Ranking sperm cells could improve the odds of in vitro fertilization. By Courtney Humphries. Technology Review. Wednesday, January 21, 2009), in particular individual deleterious genetic traits involved in a genetic disease or in a multifactorial disorder in the offspring.

Methods for separating spermatozoids into mature and immature populations (EP 1056336), into alive and dead (or apoptotic) populations (Tamer M. Said et al., "Utility of Magnetic Cell Separation as a Molecular Sperm Preparation Technique", Journal of Andrology, Vol. 29, No. 2, March/April 2008), into euploid and aneuploid populations (WO 2006/116627), or into X and Y chromosome bearing populations (WO 90/13303) have been described. Flow cytometry techniques and/or staining techniques are classically employed in this context.

The ability to identify the gametes which do not exhibit deleterious traits involved in a genetic disease or in a multifactorial disorder in the offspring, however remains an important challenge in the context of preventive medicine.

The present invention now provides a method of separating populations of gametes based on the presence or absence of a specific deleterious trait in their genome, corresponding to (an) abnormal nucleic acid sequence(s) involved in the onset, in the offspring, of a genetic disease or of a multifactorial disorder. In a typical embodiment, the method is a method of separating populations of haploid gametes (the term "haploid" being herein understood as referring to cells with a correct number of chromosomes, i.e., comprising 23 chromosomes).

### SUMMARY OF THE INVENTION

The present invention offers a novel and unique approach, in the context of preventive medicine, to avoid the transmission of a deleterious trait leading to a genetic or multifactorial disease in the offspring of a subject.

The present invention in particular provides a solution to limit or avoid preimplantation genetic diagnosis, invasive testing during pregnancy, as well as pregnancy interruption or termination.

A method of separating gametes of a subject, which method comprises discriminating a first population of gametes containing an abnormal nucleic acid sequence involved in a genetic disease or in a multifactorial disorder in the offspring of the subject, from a second population of gametes which does not contain said abnormal nucleic acid sequence, is herein described. The method may further comprise a step of recovering the second population of gametes.

The present invention further concerns the population of gametes recovered or produced by the herein described method.

A particular object of the invention relates to the use, for separating the gametes of a subject of at least one substance capable of discriminating (either in the sperm sample or on the follicular fluid containing oocytes) one or more populations of gametes containing or carrying an abnormal nucleic acid sequence involved in a genetic disorder or in a multifactorial disorder in the offspring of the subject, from one or more other populations of gametes which do not carry said abnormal nucleic acid sequence.

Another embodiment of the present invention further concerns a kit comprising any one or more of the herein-described products.

### LEGENDS TO THE FIGURES

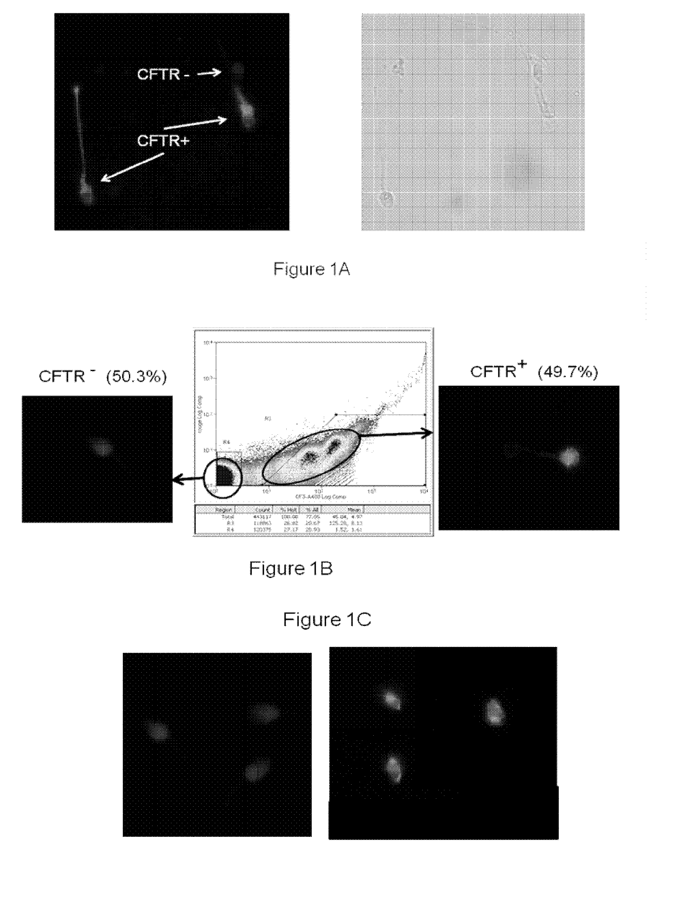

**FIGURE 1A** shows ejaculated sperm cells from a healthy carrier, heterozygous for the ΔF508 mutation on the CFTR gene. Sperm was centrifuged on a density gradient made of SupraSperm in order to obtain the high mobility fraction composed of viable spermatozoids. Cells were then labelled with an anti-CFTR monoclonal antibody (the discriminating substance) and stained to visualize the nuclei (see the experimental section). They were then observed under a fluorescence microscope. Green fluorescence reveals the presence of the CFTR protein. Blue fluorescence reveals the presence of the nuclei. The sperm of the healthy carrier, heterozygous for the ΔF508 mutation, contains both types of spermatozoids: those expressing the CFTR protein (green and blue fluorescences) and those which do not express the CFTR protein (blue fluorescence only).
**FIGURE 1B** shows the separation, using fluorescence activated cell sorting, of the two types of sperm cells present in the sperm of a heterozygous carrier of the ΔF508 mutation on the CFTR gene: the CFTR+ cells (cells expressing the CFTR protein) and CFTR- cells (cells which do not express the CFTR protein). Sperm cells recovered from the high mobility fraction after centrifugation in a density gradient (see legend of Figure 1A) were incubated with a first anti-CFTR monoclonal antibody and with a second antibody directed against said first antibody. Sperm cells were then separated by fluorescence activated cell sorting (see the experimental section).
**FIGURE 1C** shows the expression (or absence of expression) of the CFTR protein in the sperm cells obtained from each of the two fractions observed on Figure 1B. Sperm cells recovered from the fluorescence activated cell sorter were stained for visualization of the nuclei and observed under the fluorescence microscope. Green fluorescence reveals the presence of the CFTR protein. Blue fluorescence reveals the presence of the nuclei. The two populations of cells recovered from the cell sorter differ in that one population expresses the CFTR protein and shows green fluorescence (CFTR+ cells, right side picture) while the second population does not express such protein and shows only blue (nuclei) fluorescence (CFTR- cells, left side picture).

### DETAILED DESCRIPTION OF THE INVENTION

The DNA sequence of any gene can vary among individuals in the population. The various forms of a gene are called alleles, and diploid organisms generally have two alleles for each gene, one on each of the two homologous chromosomes on which the gene is present. In diploid organisms, the alleles are inherited from the individual's parents, one from the male parent and one from the female.

The term "zygosity" refers to the similarity of alleles of a gene for a trait (inherited characteristic) in an organism. The words "homozygous", "heterozygous", and "hemizygous" are used to describe the genotype of a diploid organism at a single locus on the DNA. Simply stated, "homozygous" describes a genotype consisting of two identical alleles at a given locus, "heterozygous" describes a genotype consisting of two different alleles at a locus, "hemizygous" describes a genotype consisting of only a single copy of a particular gene in an otherwise diploid organism, and "nullizygous" refers to an otherwise diploid organism in which both copies of the gene are missing. An individual that is "homozygous dominant" for a particular trait, carries two copies of the allele that codes for the dominant trait. This allele is often called the "dominant allele".

An individual that is "homozygous recessive" for a particular trait, carries two copies of the allele that codes for the recessive trait. This allele is often called the "recessive allele".

If a particular trait in an organism is determined by dominance, a heterozygote organism will express only the trait coded by the dominant allele and the trait coded by the recessive allele will not be present.

The notion of 'dominant' or 'recessive', whenever applied to a particular allele, is relative from one allele to the other. This means that a specific allele is 'dominant' with respect to a second specific allele (which is therefore 'recessive' towards the first allele) if the second allele is not expressed in the phenotype of the heterozygous organism that carries those two alleles. Thus, the so-called normal (non mutant) allele of a gene is 'dominant' with respect to a 'recessive' allele of that gene; but may be 'recessive' with respect to a 'dominant' allele of that same gene.

During the process of formation of the gametes and as a result of the meiosis that takes place in the process, the two alleles of each gene are physically separated into two different gametes. As a result, each particular gamete (whether a spermatozoid or an oocyte) carries a unique combination of the alleles coming from the different genes present in the genome but, among them, it carries only one of the two alleles for each of those genes. Gametes are therefore 'haploid' cells. When two gametes (one spermatozoid and one oocyte) fuse together in the process of fertilization, the resulting cell, the zygote, is a 'diploid' cell. The zygote receives, for each of the genes in the genome, one allele from the spermatozoid (from the father) and one allele from the oocyte (from the mother).

The oocyte (or ovum or ocyte or egg) is the haploid cell that is the female gamete. The human oocyte comprises or carries 23 chromosomes.

The spermatozoid, also called spermatozoo, is the haploid cell that is the male gamete. The human spermatozoid comprises 23 chromosomes.

Oocytes and spermatozoids cannot divide. Spermatozoids and oocytes further have a limited life span.

The spermatozoid joins an oocyte and their fusion forms a totipotent zygote comprising 46 chromosomes, i.e., a cell with a complete set of chromosomes, with the potential to develop into a new organism (offspring or progeny). Each of the spermatozoid and oocyte contributes half of the genetic information to the diploid offspring.

In mammals, the sex of the offspring is determined by the spermatozoid: a spermatozoid bearing a Y chromosome will lead to a male (XY) offspring, while one bearing an X chromosome will lead to a female (XX) offspring (the oocyte or ovum always provides an X chromosome).

The present invention provides methods of separating or selecting gametes (spermatozoids or oocytes) of a subject.

In an embodiment, the method comprises discriminating at least one population of gametes containing at least one abnormal or altered nucleic acid sequence involved in a genetic disease or in a multifactorial disorder in the offspring of the subject, from another population of gametes which does not comprise said abnormal nucleic acid sequence.

The herein described methods may be performed *in vitro, ex vivo* or *in vivo* (in particular in the female reproductive tract).

In the context of the present invention, the subject is a diploid organism. The subject is preferably a mammal, and in particular is a human being.

The population of gametes herein identified as 'first population' contains a majority of gametes containing or comprising (i.e., carrying or expressing) an abnormal or altered nucleic acid sequence involved in at least one particular genetic disease or multifactorial disorder in the offspring of the subject.

The 'first population' of gametes may itself comprise sub-populations of gametes, each sub-population containing gametes carrying different kinds, or expressing different levels, of abnormal or altered nucleic acid sequence(s), said sequence(s) being involved at least one particular genetic disease or multifactorial disorder in the offspring of the subject.

The population of gametes herein identified as 'second population' contains a majority of gametes which do not contain or comprise (i.e., carry or express) the abnormal or altered nucleic acid sequence(s) carried or expressed by the gametes of the 'first population'.

The 'second population' of gametes may itself comprise sub-populations of gametes, each sub-population containing gametes which do not comprise or do not express the abnormal or altered nucleic acid sequence(s) contained by the 'first population'.

In a particular embodiment, the 'second population' of gametes does not comprise more than 40%, 30%, 20% or 10% of abnormal gametes, i.e., of gametes expressing an abnormal or altered nucleic acid sequence involved in at least one particular genetic disease or multifactorial disorder in the offspring of the subject, preferably not more than 5%, even more preferably not more than 1%. In a particularly preferred embodiment, the 'second population' of gametes does not comprise abnormal gametes.

In a preferred embodiment, the methods herein described further comprise a step of recovering the 'second population' of gametes as further explained below.

The 'second population' may then advantageously be used as a fertilizing sample, or to prepare such a fertilizing sample, in the context of an *in vitro, ex vivo* or *in vivo* assisted reproductive technique.

In a particular embodiment, a method of fertilizing a female subject comprising a step of administering to said subject a population of gametes, in particular a population herein identified as 'second population', which has been recovered from a method of separating gametes as herein described, or a fertilizing sample (as mentioned previously), is herein disclosed.

In the context of the present invention, the expression "abnormal or altered nucleic acid sequence involved in the a genetic disease or in a multifactorial disorder in the offspring of the subject", refers to a nucleic acid sequence [deoxyribonucleic acid (DNA) or ribonucleic acid (RNA)] differing from the normal sequence in that its expression, or non-expression, predisposes to (i.e., significantly enhances the risk of developing) or is responsible for the onset of a genetic disease, or predisposes to a multifactorial disorder.

The normal nucleic acid sequence is a sequence which is not involved in the onset of a specific genetic disease or multifactorial disorder in the offspring of a subject. Preferably, the normal nucleic acid sequence is not involved in the expression of an abnormal trait (as herein defined) in the offspring of a subject.

In a typical embodiment of the present invention, the normal nucleic acid sequence is a naturally existing sequence or an artificial sequence (i.e. a particular sequence obtained by genetic engineering). Preferably, the normal nucleic acid sequence is a naturally existing wild-type sequence.

In the context of the present invention, an abnormal or altered nucleic acid sequence is one which is different in some manner from the previously defined normal nucleic acid sequence and is, as a consequence of said difference, involved in the onset in particular of a specific genetic disease or multifactorial disorder, in the offspring of a subject.

Such abnormality or alteration includes, but is not necessarily limited to, a mutation in the nucleic acid sequence.

The mutation may be selected from a deletion of one or more nucleotides, an addition of one or more nucleotides, an insertion of one or more nucleotides, a duplication of one or more nucleotides, a splice site mutation, an inversion, the substitution of one or more nucleotides by natural nucleotides or nucleotidic or non nucleotidic analogues thereof (i.e., exhibiting the same function), and combination thereof.

In a particular embodiment, the mutation is a point mutation (e.g., a single nucleotide polymorphism, also named SNP) or a mutation of at least two consecutive nucleotides. In another embodiment, the mutation results from the insertion of a sequence of viral, bacterial, or mammalian origin, into the gamete genome. Sequences of viral origin may be for example retroviral, lentiviral, adenoviral, or adeno-associated viral sequences.

It will be understood that the above mentioned types of mutations or abnormalities can co-exist in the same nucleic acid sequence.

In a particular embodiment, the abnormal nucleic acid sequence may consist in, or be present in, a coding region.

In another particular embodiment, the abnormal nucleic acid sequence may consist in, or be present in, a non-coding region of the genome, such as an intra-genic sequence, for example an intron sequence, an inter-genic sequence (i.e., a sequence located between two genes), a regulatory sequence (including a sequence regulating splicing).

Mutations in a coding or non-coding region may lead to the abnormal transcription of DNA, to the abnormal splicing of RNA, the abnormal folding or de-folding of a DNA or RNA molecule, to an abnormal base-pairing, replication, recombination, repair and/or ability to be repaired or recombined of a DNA or RNA molecule, or to the absence of binding or to the abnormal binding of molecules (for example the abnormal binding of DNA and RNA molecules, or of the encoded protein, with a molecule selected from DNA, RNA, another protein, a cofactor, an ion, a substrate, etc.).

Mutations in a coding region may in particular lead to a change in the amino acid sequence of the encoded polypeptide, e.g., an amino acid substitution, a frame-shift mutation and/or a truncated polypeptide sequence, for example a sequence deprived of a particular amino acid domain or sub-domain.

In a further particular embodiment, the abnormal nucleic acid sequence may consist in, or be in, a sequence regulating the expression of a gene (i.e. a non-coding region), such as for example a promoter sequence, an enhancer sequence, a poly A sequence, a sequence regulating the transcription of DNA into RNA, a sequence regulating the RNA maturation (in particular a splicing-controlling sequence), the RNA translocation, the RNA processing, the RNA translation and/or the RNA susceptibility to RNAses, etc.

Mutations in a non-coding region, in a regulatory region for example, may more particularly lead for example to the abnormal transcription of a DNA molecule, to an abnormal expression (up-regulation or down-regulation) or an abnormal localization of the expression of a RNA molecule and/or of a protein, to the change of a constitutive expression into an inducible or regulated expression, to the abnormal folding of a DNA or RNA molecule, to an abnormal base-pairing, replication, recombination, repair and/or ability to be repaired or recombined of a DNA or RNA molecule, or to the abnormal binding of molecules (for example the abnormal binding of DNA and RNA molecules, or of the encoded protein, with a molecule selected from DNA, RNA, another protein, a cofactor, an ion, a substrate, etc.).

In a preferred embodiment, an abnormal nucleic acid sequence as herein described may be responsible for an absent or abnormal expression of the protein encoded by the normal version (as defined previously) of the nucleic acid sequence.

A gamete comprising an abnormal nucleic acid sequence is herein also identified as a gamete comprising an abnormal genotype, in particular an abnormal genotype involved in a genetic disease or a multifactorial disorder in the offspring.

An abnormal protein is a protein or polypeptide whose presence, absence, altered expression (altered property or function) may create, lead to or induce an abnormal phenotype (such as a pathologic or diseased phenotype or an abnormal activity or behaviour) in a cell or tissue, during a short or long period of the person's (progeny's) life.

An abnormal protein is a protein or polypeptide whose sequence, amount, configuration, maturation, stability, half-life, secretion, turn over, immunogenicity, pharmacodynamic and/or pharmacokinetic properties, ability to bind another molecule, expression level, in particular expression level over time, expression pattern in tissues, expression pattern in a sub-cellular body and/or biological activity for example is (are) different from the corresponding feature(s) of the normal protein.

Typically, the abnormal protein is not functional or only partially functional.

A genetic disease or disorder is a disease or disorder resulting from, or at least partly caused by, at least one abnormal nucleic acid sequence as previously defined.

In the context of the present invention, the genetic disorder is preferably a highly penetrant genetic disorder which means that a relatively high proportion of those who inherit the abnormal nucleic acid sequence go on to develop the disease, in particular an incapacitating disease (for example a premature disability), in particular a disease for which the therapeutic treatment is limited or does not exist, more particularly a disease causing early or premature death.

In the context of the present invention, the genetic disorder or disease may be selected from a complex genetic disorder (likely associated with the effects of multiple abnormal nucleic acid sequences), in particular a polygenic disorder; a single gene disorder; an autosomal dominant disorder; an autosomal recessive disorder; a X-linked dominant disorder; a X-linked recessive disorder; and a Y-linked disorder. More specifically, the genetic disorder may be selected from the diseases mentioned in the present description. Preferably, the genetic disorder is not a genetic disorder caused by aneuploidy, i.e., by an abnormal number of chromosome (extra and/or missing chromosome(s)).

A single gene disorder is the result of a single mutated gene. There are estimated to be over 4000 human diseases caused by single gene defects. Single gene disorders can be passed on to subsequent generations in several ways. Genomic imprinting and uniparental disomy (UPD) may affect inheritance patterns.

For the vast majority of autosomal genes, expression occurs from both alleles simultaneously. In mammals however, a small proportion (<1%) of genes are imprinted, meaning that gene expression occurs from only one allele. The expressed allele is dependent upon its parental origin. For example, the gene encoding Insulin-like growth factor 2 (IGF2/Igf2) is only expressed from the allele inherited from the father. Appropriate expression of imprinted genes is important for normal development, with numerous genetic diseases associated with imprinting defects including Beckwith-Wiedemann syndrome, Silver-Russell Syndrome, Angelman Syndrome and Prader-Willi Syndrome.

Uniparental disomy (UPD) occurs when a person receives two copies of a chromosome, or part of a chromosome, from one parent and no copy from the other parent.

Only one mutated copy of the gene will be necessary for a person to be affected by an autosomal dominant disorder. Each affected person usually has one affected parent. There is a 50% chance that a child will inherit the mutated gene. Examples of this type of disorder are Huntington's disease (prevalence: 1/2500), Neurofibromatosis 1, Marfan Syndrome (prevalence: 1/20000), Hereditary nonpolyposis colorectal cancer, and Hereditary multiple exostoses, which is a highly penetrant autosomal dominant disorder.

Two copies of the gene must be mutated for a person to be affected by an autosomal recessive disorder. An affected person usually has unaffected parents who each carry a single copy of the mutated gene (and are referred to as carriers). Two unaffected people who each carry one copy of the mutated gene have a 25% chance with each pregnancy of having a child affected by the disorder. Examples of this type of disorder are cystic fibrosis (prevalence: 1/2000), sickle-cell disease (or sickle-cell anemia or drepanocytosis; prevalence: 1/625 in the African American population), Tay-Sachs disease (prevalence: 1/3000 in the American Jews population), phenylketonuria (prevalence: 1/12000), mucopolysaccharidoses (prevalence: 1/25000), Niemann-Pick disease, spinal muscular atrophy (SMA).

X-linked dominant disorders are caused by an abnormal nucleic acid sequence on the X chromosome. Males and females are both affected in these disorders, with males typically being more severely affected than females. Examples of this type of disorder are X-linked hypophosphatemia, focal dermal hypoplasia, Aicardi syndrome, Incontinentia Pigmenti, Rett syndrome, CHILD syndrome, Lujan-Fryns syndrome. Some X-linked dominant conditions such as Rett syndrome, Incontinentia Pigmenti type 2 and Aicardi Syndrome are usually fatal in males either *in utero* or shortly after birth, and are therefore predominantly seen in females. Exceptions to this finding are extremely rare cases in which boys with Klinefelter Syndrome (47,XXY) also inherit an X-linked dominant condition and exhibit symptoms more similar to those of a female in terms of disease severity. The risk of passing on an X-linked dominant disorder differs between men and women. The sons of a man with an X-linked dominant disorder will all be unaffected (since they receive their father's Y chromosome), and his daughters will all inherit the condition. A woman with an X-linked dominant disorder has a 50% chance of having an affected fetus with each pregnancy, although it should be noted that in cases such as Incontinentia Pigmenti only female offspring are generally viable. In addition, although these conditions do not alter fertility per se, individuals with Rett syndrome or Aicardi syndrome rarely reproduce.

X-linked recessive disorders are also caused by an abnormal nucleic acid sequence on the X chromosome. Males are more frequently affected than females, and the chance of passing on the disorder differs between men and women. The sons of a man with an X-linked recessive disorder will not be affected, and his daughters will carry one copy of the mutated gene. A woman who is a carrier of an X-linked recessive disorder (X^{R}X^{r}) has a 50% chance of having sons who are affected and a 50% chance of having daughters who carry one copy of the mutated gene and are therefore carriers. Examples of this type of disorder are hemophilias, in particular haemophilia A (prevalence: 1/10000) and haemophilia B (prevalence: 1/60000); muscular dystrophies, such as Duchenne muscular dystrophy (DMD - prevalence: 1/7000) and Becker muscular dystrophy.

Other examples of X-linked disorders are Chronic granulomatous disease (CYBB), Wiskott-Aldrich syndrome, X-linked severe combined immunodeficiency, X-linked agammaglobulinemia, Hyper-IgM syndrome type 1, X-linked lymphoproliferative disease, X-linked sideroblastic anemia, Androgen insensitivity syndrome, Kennedy disease, Kallmann syndrome, X-linked adrenal hypoplasia, ornithine transcarbamylase deficiency, oculocerebrorenal syndrome, Glucose-6-phosphate dehydrogenase deficiency, pyruvate dehydrogenase deficiency, Danon disease, glycogen storage disease Type IIb, Fabry's disease, Hunter syndrome, Lesch-Nyhan syndrome, Barth syndrome, McLeod syndrome, Simpson-Golabi-Behmel syndrome, Alport syndrome, Dent's disease, X-linked nephrogenic diabetes insipidus, centronuclear myopathy, Conradi-Hünermann syndrome, dyskeratosis congenita, hypohidrotic ectodermal dysplasia, X-linked ichthyosis, X-linked endothelial corneal dystrophy, Charcot-Marie-Tooth disease, Pelizaeus-Merzbacher disease, Choroideremia, Color blindness (red and green) Ocular albinism, Norrie disease, X-Linked mental retardation, Coffin-Lowry syndrome, Fragile X syndrome, MASA syndrome, X-linked alpha thalassemia, mental retardation syndrome, X-linked mental retardation syndrome and Menkes disease.

A multifactorial disorder is a disorder or disease which is caused in part by at least one abnormal nucleic acid sequence, as previously defined, and in part by at least one environmental factor. In other words, environmental factors may determine the development of disease in those individuals genetically predisposed to a particular condition.

Physical and mental stress, diet, exposure to toxins, physical inactivity, pathogens, smoking, radiations and chemicals are examples of common environmental factors which may be involved in the onset of a multifactorial disorder.

Complex or multifactorial disorders include cardiovascular diseases, asthma, diabetes, epilepsy, hypertension, cancers, metabolic diseases, in particular inflammatory bowel disease and obesity; neurological diseases, in particular autism, manic depression and schizophrenia; immunological diseases, in particular autoimmune diseases such as multiple sclerosis; and hematological disease.

Developmental abnormalities are also included in this category, such as cleft lip/palate, congenital heart defects and neural tube defects.

The incidence of any multifactorial disorder is dependent on a balance of risks. There is a balance between gene (alleles) variants and environmental factors which may predispose or, on the contrary, protect the subject toward a particular disease. Multifactorial disorders are generally difficult to treat and may cause premature disability or death. Even if they do not have a clear-cut pattern of inheritance, they often cluster in families.

As indicated previously, the herein described methods of separating gametes of a subject comprise discriminating populations of gametes.

The discrimination may be performed by using a physical discriminating method.

Such a method may be selected from a flow cytometry technique (in particular fluorescence activated cell sorting or magnetic activated cell sorting); a technique based on the analysis of cell movement, cell migration (such as chemotaxis), cell sedimentation (in particular centrifugation, density or chemical gradients); a technique based on an irradiation (in particular a technique using fluorescence, optical microscopy); a technique based on cell binding (in particular binding to an artificial or biological particle or matrix, or to a chromatographic support) or on cell retention or immobilization; a technique using the permeation properties of the cell membrane (in particular swelling, lysis); a technique based on micromanipulation; a technique using electricity (in particular electroporation, electro-shocking) and any combinations thereof.

The specific physical discriminating method(s) which can be used in the context of the present invention will be chosen based on several criteria including in particular (i) the nature of the disease, (ii) the gene(s) and nucleic acid sequence(s) involved in the onset of said disease and (iii) the abnormal phenotype caused in part by at least one abnormal nucleic acid sequence.

For example, if the abnormal nucleic acid sequence encodes an abnormal epitope of a transmenbrane protein, this abnormal epitope may be detected by differential binding to a specific monoclonal antibody unable to bind the normal epitope.

Inversely, the abnormal epitope may be detected by differential binding of a specific antibody able to bind the normal sequence but unable to bind the abnormal sequence.

In another example, the abnormal sequence may be responsible for the non expression of the protein in the membrane. In this particular context, a monoclonal antibody able to bind the normal protein will be unable to bind it as said protein is absent from the membrane. The non binding therefore reveals the presence of an abnormal sequence.

In a further example, the normal sequence codes for a cytoplasmic protein involved in reticular or cytoskeleton structures in the cell, for example in maintaining the anchorage of transmembrane proteins and protein complexes; and the abnormal sequence may be responsible for the abnormal formation of a transmembrane protein complexe. In this example, the abnormal sequence may be detected indirectly by differential binding of a specific monoclonal antibody able to bind the normal transmembrane complex, but unable to bind an abnormal or absent complex.

In the previous examples, and in the context of the present invention, the gametes expressing the epitope binding the specific monoclonal antibody may be simultaneously discriminated and recovered using for example a fluorescence activated cell sorter or a magnetic sorting technique according to protocols well known by the person skilled in the art.

In another example, the abnormal nucleic acid sequence changes the metabolism of the gamete. Such a change in the metabolism may, for example, modify the spermatozoid mobility. The two populations of spermatozoids can be discriminated using a method, according to the present invention, comprising a step which may consists for example in a centrifugation in density gradients or in a migration-based physical protocol known by the person skilled in the art.

In another example, the abnormal nucleic acid sequence encodes for an abnormal membrane receptor on the surface of the gamete, such abnormal receptor being unable to recognize its ligand. The abnormal sequence may thus be detected by differential binding of such a membrane receptor to the specific ligand.

In this example, and according to the present invention, the gametes expressing the normal receptor, which bind the specific ligand, can be simultaneously discriminated and recovered using for example a fluorescence sorting or magnetic sorting technique, or using a technique allowing the binding of the receptor to a particle, a matrix or a support well known by the person skilled in the art.

In a further example, the abnormal nucleic acid sequence changes the metabolism of the gamete in such a way that the pattern of at least one extracellular glycoprotein (several and diverse glycoproteins are present in the gametes) is altered. Such a change may, for example, affect the binding of a specific protein to the surface of the gamete.

In this example, and according to the present invention, the gametes expressing the normal pattern of the extracellular glycoprotein, may be simultaneously discriminated and recovered using for example a fluorescence sorting or magnetic sorting technique, or using a technique allowing the binding of the extracellular glycoprotein to a particle, a matrix or a support well known by the person skilled in the art.

The discrimination may be performed by contacting the gametes of the subject with at least one discriminating substance capable of (i) altering or destroying the first population of gametes (or subpopulations of said first population), or of (ii) allowing the identification of the first or of the second population of gametes (or subpopulations thereof), thereby discriminating the first and second populations of gametes (or subpopulations thereof).

In the context of the present invention, the discriminating substance is a substance allowing the distinction of gametes containing (carrying and/or expressing) an abnormal nucleic acid sequence involved in a genetic disease or a multifactorial disorder in the offspring of the subject, from gametes which do not contain said abnormal nucleic acid sequence.

The discriminating substance is a chemical or a biological substance of natural or artificial (engineered or synthetic) origin.

The discriminating substance may be a molecule or a composition of molecules. It can be for example a chemical (inorganic or organic compound) or a biological compound. The discriminating substance may be an amino acid sequence (for example a peptide, a polypeptide, a protein) or a nucleic acid sequence, or a combination thereof. The discriminating substance may more particularly be an aptamer, an antibody, a cytokine, an hormone, a growth factor, or a functional fragment thereof. The discriminating substance may further be a virus, a cell organelle or a cell fragment.

The discriminating substance may act as:
- an antibody capable of binding a specific cellular epitope;
- a substrate, a cofactor, an inhibitor or an activator of a cellular enzyme thus altering the metabolism of the gamete;
- an hormone, a cytokine or a growth factor, thus altering the cellular activity of the gametes;
- a ligand for a specific receptor, protein or glycoprotein, thus triggering a particular cellular response in the gamete;
- an inducer of a cell signalling pathway, thus changing the activation status of the gamete (preceding fertilization), or the apoptotic or differentiation status thereof;
- an inhibitor or activator of cellular or molecular processing of the DNA or the RNA molecule (replication, repair, transcription, maturation, splicing, translation) thus affecting the expression of such a molecule in the gamete.

The discriminating substance may further control or affect the exchanges of ions, metabolites or water through the plasma membrane, thus changing the electric membrane potential or the hydration status of the gametes.

Similarly to the specific physical discriminating methods mentioned previously, the specific discriminating substance which can be used in the context of the present invention will be chosen based on several criteria including in particular (i) the nature of the disease, (ii) the gene(s) and nucleic acid sequence(s) involved in the onset of said disease and (iii) the abnormal phenotype caused in part by at least one abnormal nucleic acid sequence.

For example, if the altered or abnormal sequence produces changes in the cellular metabolism such that the cell cannot regulate the intracellular osmolarity, the presence of such an abnormal sequence may be detected by an increased sensitivity of the cell to swelling and to osmotic shock. In this example, and according to the present invention, the gametes carrying the abnormal sequence will be lysed in the presence of a discriminating substance able to stimulate water and/or ion movements across the cell membrane and thereby changing osmolarity This is the case for spermatozoid cells expressing an abnormal CFTR sequence when said spermatozoids are incubated in the presence of adrenaline or of an analogue of adrenaline.

In another example, if the altered or abnormal sequence decreases the ability of a specific cell protease to degrade a specific substrat, the gametes expressing such an altered sequence (for example an altered calpain 3 sequence) can be discriminated, using a method according to the present invention, by their inability to digest this specific substrate used as a discriminating substance, whose degradation can be monitored. In this example, and according to the present invention, a synthetic polypeptide which becomes fluorescent (or alternatively, which looses fluorescence) upon proteolytic digestion, rendering fluorescent (or non-fluorescent) the gametes expressing the normal sequence, may be used as discriminating substance. This may be the case for spermatozoid cells expressing abnormal sequences of the calpain 3 gene (the gene related to LGMD2A) when incubated in the presence of the appropriate polypeptide substrates.

In a further example, if the abnormal sequence alters the level of expression of an enzyme whose activity may affect the mobility of the cell, the gametes expressing such an abnormal sequence may be discriminated by their inability to respond to an inhibitor or an activator (including the substrate) of such an enzyme.

In this example, and according to the present invention, the mobility of a spermatozoid cell expressing an abnormal sequence of the eNOS gene (the gene for endothelial nitric oxyde synthase) will not be modified when incubated in the presence of a discriminating substance such as the eNOS substrate (L-arginine) or an inhibitor of eNOS as further detailed below. In other words, in a sample, the mobility of spermatozoid cells expressing a normal sequence of the eNOS gene will be modified when incubated in the presence of such a discriminating substance (contrary to the spermatozoid cells expressing the abnormal sequence).

In another example, if the abnormal sequence alters the level of expression of a transmembrane protein, the gametes expressing such an abnormal sequence may be discriminated by their inability to bind a monoclonal antibody directed against this protein. In this example, and according to the present invention, the spermatozoid cells expressing an abnormal sequence of the CFTR gene responsible for the absence or for a decreased expression of the CFTR in the cell membrane, will not be able to bind to an anti-CFTR monoclonal antibody.

In a further example, if the abnormal sequence alters the level of expression of an intracellular protein involved in the architecture of the cytoskeleton or in its relation with proteins of the matrix, the gametes expressing such an abnormal sequence may be discriminated by their inability to bind a monoclonal antibody directed against a protein whose presence, structure or location may be altered by the disrupted cytoskeleton.

In this example, and according to the present invention, the spermatozoid cells expressing an abnormal sequence of the Dystrophin gene will not be able to bind to such a kind of monoclonal antibody.

In a particular example of the present invention, the abnormal nucleic acid sequence present in the gametes of the first population is in the gene encoding the cystic fibrosis transmembrane conductance regulator (CFTR), and the genetic disease is cystic fibrosis.

Cystic fibrosis (also known as CF or mucoviscidosis) is a devastating human autosomal recessive genetic disorder appearing at a frequency of about 1/2000 births among Caucasians. This disease affects the entire body, causing progressive disability and often, early death.

It is characterized by a deficient electrolyte transport through epithelia, giving rise to abnormalities in airway, sweat gland, pancreas and gonadal functions. Respiratory insufficiency, ultimately related to a deficient secretion of Cl- into the luminal mucus by airway epithelium cells, is the most serious symptom and results from frequent lung infections that are treated, though not cured, by antibiotics and other medications. Ultimately, lung transplantation is often necessary as cystic fibrosis worsens.

CF is caused by a mutation in the cystic fibrosis transmembrane conductance regulator (CFTR) gene. This gene has been cloned and characterized [Kerem, B.-S., Rommens, J.M., Buchanan, J.A. et al. Identification of the cystic fibrosis gene: genetic analysis. Science 245, 1073-1080 (1989); Riordan, J.K., Rommens, J.M., Kerem, B.-S. et al., Identification of the cystic fibrosis gene: cloning and characterization of complementary DNA. Science 245, 1066-1073 (1989); Rommens, J.M., Ianuzzi, M.C., Kerem, B.-S. et al., Identification of the cystic fibrosis gene: chromosome walking and jumping. Science 245, 1059-1065 (1989)].

The *CFTR* gene which is found at the q31.2 locus of chromosome 7, is 230,000 base pairs long, and creates a protein that is 1,480 amino acids long (see the NCBI Reference CFTR gene Sequence: NG_016465.1; NCBI Reference CFTR mRNA Sequence: NM_000492.3; Swiss-Prot reference CFTR protein sequence: P13569.3, herein identified as SEQ ID NO:1).

The product of this gene (the CFTR) is a chloride ion channel important in creating physiological functional sweat, digestive juices and epithelial mucus.

The most common mutation, ΔF508, is a deletion (Δ) of three nucleotides that results in a loss of the amino acid phenylalanine (F) at the 508th (508) position on the protein. This mutation accounts for two-thirds of CF cases worldwide and 90% of cases in the United States; however, there are over 1,400 other mutations that can produce CF (Bobadilla JL, Macek M, Fine JP, Farrell PM (June 2002). "Cystic fibrosis: a worldwide analysis of CFTR mutations--correlation with incidence data and application to screening". Hum. Mutat. 19 (6): 575-606.). Although most people without CF have two working copies (alleles) of the CFTR gene, only one is needed to prevent cystic fibrosis. CF develops when neither allele can produce a functional CFTR protein. A typical abnormal nucleic acid sequence according to the present invention comprises the ΔF508 mutation.

Couples who are pregnant or who are planning a pregnancy can themselves be tested for CFTR gene mutations to determine the degree of risk that their child will be born with cystic fibrosis. Testing is typically performed first on one or both parents and, if the risk of CF is found to be real, testing on the fetus can then be performed.

As indicated previously, CF can result from more than a thousand different mutations such as G85E, R117H, R334W, R347P, A455E, 508delF (ΔF508), 507delI, G542X, S549N, G551D, R553X, R560T, 621+1G->T, 711+1G->T, 1078delT, R1162X, W1282X, N1303K, 1717-1G->T, 1898+1G->A, 2184delA, 2789+5G->A, 3659delC, and 3849+10kbC->T. Typically the abnormal nucleic acid sequence is a CFTR gene with a mutation selected from ΔF508, G542X, G551D, N1303K, W1282X. It is however not possible to test for each one.

Blood is tested for the most common mutations such as ΔF508. Most commercially available tests look for 32 or fewer different mutations. If a family has a known uncommon mutation, specific screening for that mutation can be performed. However, because not all known mutations are found on current tests, a negative screen does not guarantee that a child will not have CF [Elias S, Annas GJ, Simpson JL (April 1991). "Carrier screening for cystic fibrosis: implications for obstetric and gynecologic practice". Am. J. Obstet. Gynecol. 164 (4): 1077-83].

Couples who are at high risk for having a child with CF will often opt to perform further testing before or during pregnancy. *In vitro* fertilization with preimplantation genetic diagnosis offers the possibility to examine the embryo prior to its placement into the uterus. The test, performed three days after fertilization, looks for the presence of abnormal CF genes. If two mutated CFTR genes are identified, the embryo is not used for embryo transfer and an embryo with at least one normal gene is implanted.

During pregnancy, testing can be performed on the placenta (chorionic villus sampling) or the fluid around the fetus (amniocentesis). However, chorionic villus sampling has a risk of fetal death of 1 in 100 and amniocentesis of 1 in 200 so the benefits must be determined to outweigh these risks prior to going forward with testing.

The present invention offers a solution to avoid practising preimplantation genetic diagnosis or testing during pregnancy and is more efficient than said techniques in that the separation does not depend on the specific mutation in the abnormal *CFTR* nucleic acid sequence involved in cystic fibrosis but simply on its effect on the CFTR protein.

The separation methods herein described advantageously comprise a step of contacting the gametes of the subject with a discriminating substance capable of destroying the first population of gametes (or a subpopulation thereof), as previously defined, i.e., the population of spermatozoids comprising an abnormal nucleic acid sequence in the gene encoding CFTR.

Such a discriminating substance may be selected for example from a bronchodilator, preferably epinephrine (also called adrenaline), and a vasodilator, preferably forskolin. The discriminating substance may further be a functional analogue ((+) epinephrine isoform; (-) epinephrine isoform; a cyclohexyl or 3-cyclohexenyl analog of (±)-epinephrine; a sympathomimetic agent; a vasoconstrictor agent; an adrenergic agonist; an adrenergic-beta agonist; an adrenergic-alpha agonist; a pyridine compound analogous to epinephrine; ephedrine; tyramine; pseudoephedrine; amphetamines; isoprenaline; orciprenaline; salbutamol; terbutaline; orciprenaline; acidic epinephrine analogues derived from 1H, 3H-2,1,3-benzothiadiazole 2,2-dioxide or from trifluoromethanesulfonanilide.) of one of the previously identified substances, or a combination of said substances.

Inventors discovered that they could use the properties of these molecules in promoting the intake of water into the treated cells to create an increase in the intracellular osmotic pressure leading to cell swelling. Because of the absence of functional chloride channels in their membrane, CFTR deficient cells cannot export properly the excess of intracellular water produced by the treatment and explode by osmotic shock.

In a different embodiment, the separation method comprises a step of contacting the gametes of the subject with a discriminating substance allowing the identification of the 'first' or of the 'second population' of gametes (or of subpopulations thereof).

A discriminating substance allowing the identification of the first population of gametes (or a subpopulation thereof) is for example an anti-abnormal CFTR antibody, typically an anti-abnormal CFTR antibody directed against a CFTR fragment selected from a fragment comprising the amino acids from position 103 to position 117, 216 to 220, 881 to 911 and 1124 to 1128 of the sequence corresponding to the sequence herein identified as SEQ ID NO:1, and referenced by Swiss-Prot: under number P13569.3 (corresponding to the NCBI Reference homo sapiens cystic fibrosis transmembrane conductance regulator (ATP-binding cassette sub-family C, member 7) mRNA CFTR Sequence: NM_000492.3) or a functional analogue thereof, which selectively binds to the human abnormal CFTR protein.

A discriminating substance allowing the identification of the second population of gametes (or a subpopulation thereof) is for example an anti-CFTR antibody, or a functional analogue thereof, which selectively binds to the human normal CFTR protein on epitopes or domains of the CFTR protein that are either absent or not accessible in the gametes carrying the abnormal sequence.

Methods of making such antibodies are known in the art [see for example Köhler G et al. (1975), White A et al. (1982), Nakamura RM. Et al. (1983), Yokoyama WM. Et al. (2001) and Riechmann L et al; (1988)].

Antibodies usable in the context of the present invention are preferably labelled with one or more tags allowing for their identification, follow-up, detection and/or measurement. Tags may be selected for example from a fluorophore, a magnetic bead, an antigenic epitope, a substrate of a specific enzyme, a binding domain of a specific ligand, and any other molecule or moiety which may be detected or quantified. Antibodies usable in the context of the present invention may also be anti-antibodies used to identify, follow-up, detect and/or measure the antibodies that recognize the gametes expressing either the normal nucleic acid sequence or the abnormal nucleic acid sequence.

In another particular embodiment of the present invention, the genetic disease is a myopathy and the abnormal nucleic acid sequence present in the gametes of the first population is in the coding, in the non-coding or in the regulatory sequence of a gene selected for example from a gene encoding a calpain, a gene encoding a dystrophin, the gene encoding Wiskott-Aldrich Syndrome Protein (WASp) and the SGCG gene encoding gamma-sarcoglycan.

A myopathy is a muscular disease in which the muscle fibers do not function for any one of many reasons, resulting in muscular weakness.

Dystrophies (or muscular dystrophies abbreviated MD) are a subgroup of hereditary myopathies characterized by progressive skeletal muscle weakness, defects in muscle proteins, and the death of muscle cells and tissue.

Nine diseases including Duchenne muscular dystrophy (DMD), Becker muscular dystrophy (BMD), limb girdle muscular dystrophy or Erb's muscular dystrophy, congenital muscular dystrophy (CMD), facioscapulohumeral muscular dystrophy (FSHMD, FSHD or FSH), which is also known as Landouzy-Dejerine, Myotonic dystrophy (*dystrophia myotonica,* DM), Oculopharyngeal dystrophy (OPD, or oculopharyngeal muscular dystrophy), Distal muscular dystrophy (or distal myopathy), and Emery-Dreifuss muscular dystrophy, are always classified as muscular dystrophy but there are more than 100 diseases in total with similarities to muscular dystrophy. Most types of MD are multi-system disorders with manifestations in body systems including the heart, gastrointestinal and nervous systems, endocrine glands, skin, eyes and other organs.

The Duchenne muscular dystrophy (DMD), in particular, is a severe recessive X-linked form of muscular dystrophy characterized by rapid progression of muscle degeneration, eventually leading to loss of ambulation and death. This affliction affects one in 3500 males, making it the most prevalent of muscular dystrophies. In general, only males are afflicted, though females can be carriers. Females may be afflicted if the father is afflicted and the mother is also a carrier.

The disorder is caused by a mutation in the *DMD* gene located, in humans, on the X chromosome (Xp21). The *DMD* gene codes for the dystrophin protein, an important structural component within muscle tissue responsible for connecting the cytoskeleton of each muscle fibers to the underlying basal lamina (extracellular matrix) through a protein complex, the dystroglycan complex (DGC) which contains many subunits and is located on the cell membrane. The absence of dystrophin permits excess calcium to penetrate the sarcolemma (cell membrane). In a complex cascading process that involves several pathways and is not clearly understood, increased oxidative stress within the cell damages the sarcolemma, and eventually results in the death of the cell. Muscle fibers undergo necrosis and are ultimately replaced with adipose and connective tissue.

Symptoms usually appear in male children before age 5 and may be visible in early infancy.

The average life expectancy for patients afflicted with DMD varies from early teens to age mid 30s.

There is no known cure for Duchenne muscular dystrophy, although recent stem-cell research is showing promising vectors that may replace damaged muscle tissue. Treatment is generally aimed at controlling the onset of symptoms to maximize the quality of life.

Dystrophin is the product of the *DMD* gene, the largest known gene in the human genome, consisting of approximately 2500 nucleotides found on the X chromosome. Dystrophin has several different isoforms, from the largest (427 kDa), found in muscle cells, to the smallest (71 kDa) found in several non-muscle tissues including sperm cells.

The main role of dystrophin is to serve as a connection between actin and a number of membrane and transmembrane proteins such as syntrophin, dystrobrevins and dystroglycans. All of the isoforms, from the 427 kDa to the 71 kDa show the same basic structure, the main difference among the isoforms being the number of spectrin-like repeats. Furthermore, in the 71 kDa isoform, there is an alternate promoter.

The muscle-specific isoform of the dystrophin gene is composed of 79 exons, and DNA testing and analysis can usually identify the specific type of mutation of the exon or exons that are affected. DNA testing confirms the diagnosis in most cases. If DNA testing fails to find the mutation, a muscle biopsy test may be performed. A small sample of muscle tissue is extracted (usually with a scalpel instead of a needle) and a dye is applied that reveals the presence of dystrophin. Complete absence of the protein indicates the condition.

If one or both parents are 'carriers' of a particular condition there is a risk that their unborn child will be affected by that condition. 'Prenatal tests' are carried out during pregnancy, to try to find out if the fetus (unborn child) is affected. The tests are only available for some neuromuscular disorders. Different types of prenatal tests can be carried out after about 11 weeks of pregnancy. Chorion villus sampling (CVS) can be done at 11-14 weeks, and amniocentesis after 15 weeks, while fetal blood sampling can be done at about 18 weeks. Earlier testing would allow early termination, but it carries a slightly higher risk of miscarriage than later testing (about 2%, as opposed to 0.5%).

In the context of DMD, an abnormal nucleic acid sequence is an abnormal DMD gene, more particularly a *DMD* gene comprising a mutation responsible for the expression of an abnormal DMD protein isoform in sperm cells.

The mutation may be selected in particular from a point mutation, a deletion and a duplication.

The point mutation is typically located in section 3' of exon 55, in a splice acceptor site, in a splice donor site, in a regulatory domain and/or in a promoter region.

The point mutation of the *DMD* gene, in the context of DMD, is typically a stop mutation [in particular a nonsense mutation or a small deletion or insertion, in particular of one base pair]; a missense mutation [typically Cys3313Phe, L54R (mutation in the actin binding domain), a ZZ-cysteine-rich mutation located in the β-dystroglycan-binding region such as Cys3313Phe, D3335H, C3340Y, Glu3367Del], or a splice site mutation [IVS11-9G to A].

The mutation of the *DMD* gene may also be a deletion or a duplication, in particular a deletion or a duplication of one exon or more.

Deletions may be located in the 3' end region, in the 5' end region or in large introns of the NH2-terminus and/or rod domains. They are more particularly located in the 3' end region of the DMD gene. The deletion typically affects all or part of exons 45-52, exons 3-19, intron 44 and/or introns 50-55. The deletion is typically a frame shift inducing mutation.

Mutations may be cysteine-rich or carboxy-terminus deletions which leads to a severe DMD phenotype. For example, dystrophin may not bind to β-dystroglycan, the loss of β-dystroglycan and sarcoglycan complex from sarcolemma may also be observed. Duplications are typically located near the 5'end of the *DMD* gene. Exons 2, 6 and/or 7 are typically duplicated. Duplications may further affect an exon selected from exons 18, 22, 38, 45-55 and 51. The mean length of a duplication is of 7 exons.

The Becker muscular dystrophy (also known as benign pseudohypertrophic muscular dystrophy) is an X-linked recessive inherited disorder characterized by slowly progressive muscle weakness of the legs and pelvis.

It is a type of dystrophinopathy, which includes a spectrum of muscle diseases in which there is insufficient dystrophin produced in the muscle cells, resulting in instability in the structure of muscle cell membrane. This is caused by mutations in the dystrophin gene, which encodes the dystrophin protein. Becker muscular dystrophy is related to Duchenne muscular dystrophy in that both result from a mutation in the dystrophin gene, but in most patients with Duchenne Muscular Dystrophy no functional dystrophin is produced making DMD much more severe than BMD.

All dystrophinopathies are inherited in an X-linked recessive manner. Sons who inherit the mutation will be affected; daughters who inherit the mutation will be carriers. Men who have Becker muscular dystrophy can have children, and all their daughters are carriers, but none of the sons will inherit their father's mutation. Prenatal testing through amniocentesis or chorionic villus sampling (CVS) for pregnancies at risk is possible if the DMD mutation is found in a family member or if informative linked markers have been identified.

Becker muscular dystrophy occurs in approximately 3 to 6 in 100,000 male births, making it much less common than Duchenne muscular dystrophy. The progression of the disease is highly variable. Symptoms usually appear in men at about ages 8-25, but may sometimes begin later. Patients can lose the ability to walk as early as age 15 in the very rare severe form. Women rarely develop symptoms, but may do so due to mosaicism.

There is no known cure for Becker muscular dystrophy and treatment is aimed at control of symptoms to maximize the quality of life.

In the context of BMD, an abnormal nucleic acid sequence is an abnormal DMD gene, more particularly a *DMD* gene comprising a mutation.

The mutation may be selected in particular from a point mutation, a deletion and a duplication. The mutated nucleic acid sequence generally encodes for a partially functional dystrophin of altered sequence.

The point mutation is typically located in section 3' of exon 55, in a splice acceptor site, in a splice donor site, in a regulatory domain and/or in a promoter region.

In the *DMD* gene, the point mutation is typically a stop mutation (in particular a nonsense mutation or a small deletion or insertion, in particular of one base pair); a missense mutation [typically Asp165Val, L54R (mutation in the actin binding domain), a ZZ-cysteine-rich mutation located in the β-dystroglycan-binding region such as Cys3313Phe, D3335H, C3340Y, Glu3367Del], or a splice site mutation (IVS25+1G to C) affecting in particular cononical splice site sequences. The splice site mutation for example may generate the insertion of amino acids or pseudoexons (for example IVS25+2036A>G in intron 25 or IVS62-285A>G in intron 62) or may be responsible for a loss of function (e.g., single base substitution disrupting the invariant GT dinucleotide at the 5' end of intron 54, or splice site mutation in CpG sequences). Deletions may be located in the 3' end region, in the 5' end region or in large introns of the NH2-terminus and/or rod domains. They are more particularly located in the 3' end region of the DMD *gene.* The deletion is typically located around exons 45-52, in exons 3-19, in intron 44 and/or in introns 50-55.

Many Becker patients have in-frame mutations in the dystrophin rod domain (in particular in exons 45 and/or 53). Such mutations reduce or increase the dystrophin length.

Duplications are typically located near the 5'end of the DMD *gene.* Exons 2, 6 and/or 7 are typically duplicated. Duplications may further affect an exon selected from exons 18, 22, 38, 45-55 and 51. The mean length of a duplication is of 7 exons.

The clinical phenotype of a muscular dystrophy correlates with the amount and function of dystophin. In most patients with Duchenne Muscular Dystrophy no functional dystrophin is produced, as explained previously, making DMD much more severe than BMD.

Considering DMD in particular, in the absence of dystrophin, the reduction in sarcoglycans and other proteins in dystrophin-glycoprotrein complex and/or the dysferlin increase in cytoplasm may be observed.

In a particular embodiment of the present invention, the spermatozoid cells expressing an abnormal sequence of the dystrophin gene are not able to bind a monoclonal antibody directed against a cell surface protein such as dystrobrevin, syncoilin, synemin, sarcoglycan, dystroglycan, laminin, syntrophin, agrin or sarcospan, whose presence, structure or location is altered by the absence, or by an insufficient amount, of dystrophin responsible for the disruption of the cytoskeleton and protein of the cellular matrix.

If the myopathy is DMD or BMPD, a substance usable to discriminate spermatozoids may therefore be a monoclonal antibody, in particular a monoclonal antibody directed against a cell surface protein selected from dystrobrevin, syncoilin, synemin, sarcoglycan, dystroglycan, laminin, syntrophin, agrin and sarcospan.

The Limb-girdle muscular dystrophy (LGMD) or Erb's muscular dystrophy is an autosomal class of muscular dystrophy that is similar but distinct from Duchenne's and Becker's muscular dystrophy. Limb-girdle muscular dystrophy encompasses a large number of rare disorders.

LGMD can begin in childhood, adolescence, young adulthood or even later. The age of onset is usually between 10 and 30. Both genders are affected equally. When limb-girdle muscular dystrophy begins in childhood the progression appears to be faster and the disease more disabling. Over time (usually many years), the person with LGMD loses muscle bulk and strength. While LGMD isn't a fatal disease, it may eventually weaken the heart and lung muscles, leading to illness or death due to secondary disorders. Treatment for LGMD is primarily supportive.

LGMD may be inherited as a dominant, recessive, or X-linked genetic defect.

The "LGMD1" family is autosomal dominant, and the "LGMD2" family is autosomal recessive.

The result of the defect is that the muscles cannot properly form the proteins needed for normal muscle function. Several different proteins can be affected, and the specific protein that is absent or defective identifies the specific type of muscular dystrophy. The affected protein may be a protein selected from Myotilin, Lamin A/C (also known as LMNA), Caveolin-3, Calpain-3, Dysferlin, Gamma-sarcoglycan, Alpha-sarcoglycan, Beta-sarcoglycan, Delta-sarcoglycan, Telethonin, Tripartite motif-containing protein 32, Fukutin-related protein, Titin (also known as connectin), O-mannosyl-transferase 1, O-mannosyl-transferase 2 and O-linked-mannose beta-1,2-N-acetylglucosaminyltransferase 1.

Calpains are a family of calcium-dependent, non-lysosomal cysteine proteases expressed ubiquitously in mammals and in many other organisms. The calpain proteolytic system includes the calpain proteases, the small regulatory subunit (CAPNS1), and the endogenous calpain-specific inhibitor, calpastatin. The structural and functional diversity of calpains in the cell is reflected in their involvement in the pathogenesis of a wide range of disorders.

Calpain 3, in particular, is a major intracellular protease. It is an heterodimer consisting of a large and a small subunit. Mutations in the wild-type gene encoding human calpain 3, located on chromosome 15 [NCBI Reference Homo sapiens calpain 3 (p94) (CAPN3) gene Sequence: NG_008660.1; NCBI Reference Homo sapiens calpain 3 (p94) (CAPN3) mRNA Sequence (transcript variant 1): NM_000070.2], are associated with limb-girdle muscular dystrophies type 2A.

Typical pathogenic mutations on the Calpain 3 gene are nonsense mutations, deletions, insertions, splice site mutations, missense mutations and/or point mutations such as polymorphisms.

A mutation leading to LGMD2A is typically at least one of D77N, S86F, R118G, C137R, I162L, E217K, G222R, E226K, P319L, H334Q, Y336N, W360C, R440W, G441D, G445R, R448C, R448G, R448H, R493W, R541Q, R572Q, R572W, S606L, Q638P, R698P, D705G and D705H.

D77N for example is to be interpretated as meaning that N is substituted to D in the mutated sequence. In other words, at position 77, the normal sequence comprises the D amino acid and the abnormal one the N amino acid.

Alternate promoters and alternative splicing result in multiple homo sapiens calpain 3 transcript variants which have been isolated (see in particular NCBI Reference Sequences NM_024344.1, NM_173087.1, NM_173088.1, NM_173089.1, NM_173090.1, NM_212465.2, NR_027911.1 and GenBank reference sequences EU791850.1 and EU791851.1).

Calpain 10 has been identified as a susceptibility gene for type II diabetes mellitus and calpain 9 has been identified as a tumor suppressor for gastric cancer.

Moreover, the hyperactivation of calpains is implicated in a number of pathologies associated with altered calcium homeostasis such as Alzheimer's disease, cataract formation, as well as secondary degeneration resulting from acute cellular stress following myocardial ischemia, cerebral (neuronal) ischemia, traumatic brain injury and spinal cord injury.

It is an embodiment of the present invention to provide a method of separating gametes as previously described, wherein the abnormal nucleic acid sequence is involved, in the offspring of the subject, of LGMD as described previously, preferably of LGMD2A.

Substances usable to discriminate gametes comprising an abnormal nucleic sequence involved in the onset of LGMD2A (as previously described) may be selected, for example, from the following peptides (tagged with fluorophores): Boc-Leu-Met-CMAC [or t-butoxycarbonyl-Leu-Met-7-amino-4-chloromethylcoumarin], *Dabcyl-*TPLKSPPPSPR-*EDANS* [4-(4-dimethylaminophenylazo)benzoic acid-TPLKSPPPSPR-5-(2-aminoethylamino)naphthalene-1-sulfonic acid ; herein identified as SEQ ID NO: 5] and *Dabcyl*-TPLKSPPPSPRE(*EDANS*)R₇ [4-(4-dimethylaminophenylazo)benzoic acid-TPLKSPPPSPRE-5-(2-aminoethylamino)naphthalene-1-sulfonic acid- Arg- Arg- Arg- Arg- Arg- Arg- Arg; herein identified as SEQ ID NO: 6].

If calpain 3 is present in the gamete, the discriminating peptide is cut by the protease. The fluorophores being, as a consequence, separated, the fluorescence disappear.

On the contrary, if calpain 3 is not expressed in the gamete, then, the discriminating peptide remains the same (i.e., is not cut) and the fluorescence generated by the discriminating peptide allows the identification of said gamete.

In a further particular embodiment, the aim of the method of separating gametes according to the present invention is to discriminate a first population of gametes (or subpopulations thereof) containing an abnormal nucleic acid sequence involved in a multifactorial disorder in the offspring of the subject, from a second population of gametes (or subpopulation thereof) which does not comprise said abnormal nucleic acid sequence.

Typically, the multifactorial disorder is selected from a cardiovascular disease, a cancer, a metabolic disease, a neurological disease, an immunological disease and a haematological disease.

Most countries face high and increasing rates of cardiovascular diseases. It is the number one cause of death and disability in the United States and most European countries (data available through 2005). A large histological study (PDAY) showed vascular injury accumulates from adolescence, making primary prevention efforts necessary from childhood [Rainwater DL, McMahan CA, Malcom GT, *et al.* (Mar 1999); McGill HC, McMahan CA, Zieske AW, et al. (Aug 2000). "Associations of coronary heart disease risk factors with the intermediate lesion of atherosclerosis in youth. The Pathobiological Determinants of Atherosclerosis in Youth (PDAY) Research Group". Arterioscler Thromb Vasc Biol. 20 (8): 1998-2004.].

Cardiovascular diseases consist of a broad range of medically important conditions that occur within the cardiovascular system, involving the heart or blood vessels (arteries and veins). These diseases includes, and the present invention relates to, aneurysm, angina, atherosclerosis, cerebrovascular accident (Stroke), cerebrovascular disease, congestive heart failure (CHF), coronary artery disease, rheumatic heart disease, hypertension, coronary heart disease (CHD) manifested as myocardial infarction (MI) (heart attack) or angina (angina pectoris) and congenital cardiovascular defects. There are additional diseases of the cardiovascular system such as arrhythmias (tachycardia, atrial fibrillation or flutter), diseases of the arteries (atherosclerosis, aortic aneurysm), peripheral vascular disease, in particular peripheral arterial disease, deep vein thrombosis, and pulmonary embolism, cardiomyopathy and peripheral vascular disease (diseases of blood vessels outside the heart and brain).

Genetic background, increasing age, gender (men are at higher risk than women) and ethnical origin are major risk factors for development of a cardiovascular disease. These are considered to be non controllable risk factors. Risk factors amenable to modification or reduction through changes in lifestyle habits include: smoking tobacco products, high cholesterol and/or triglyceride blood levels, high blood pressure, physical inactivity, overweight or obesity, diabetes, etc.

HMG CoA reductase inhibitors (statins) have been shown to improve endothelium-dependent vasodilation in both coronary and peripheral arteries apart from their cholesterol lowering effect. This improved vasodilation is likely due to the up-regulation of endothelial Nitric Oxide Synthase (eNOS). eNOS is an enzyme having potent anti-atherogenic properties that include a decrease in leukocyte adhesion, platelet aggregation, and vascular smooth muscle cell growth. The mechanism by which HMG-CoA reductase inhibitors increase eNOS expression and activity occurs via stabilization of eNOS mRNA indirectly by regulating the cell cycle of vascular endothelial cells. Increased eNOS amount results in greater vasodilation, improved blood flow, and increased endothelial function, which mitigates some of the risks associated with an ailing cardiovascular system demonstrating decreased blood flow.

Large epidemiologic studies have established that the so called metabolic syndrome (defined as a set of conditions including obesity, hypertension, hyperlipidemia and insulin resistance) increases the risk for cardiovascular disease. The precise mechanisms by which these metabolic disorders increase the propensity to develop atherosclerosis are not known. However, the reduction in the bioavailability of endothelium-derived nitric oxide (eNOS) serves as a key link between metabolic disorders and cardiovascular risk. (P L Huang, eNOS, metabolic syndrome and cardiovascular disease, Trends in Endocrinology & Metabolism, Vol 20, issue 6,, 295-302, 2009).

The present invention now provides a method of separating gametes of a subject, which method comprises discriminating a first population of gametes (or subpopulation thereof) containing an abnormal nucleic acid sequence involved in a multifactorial disorder corresponding in particular to a cardiovascular disease or disorder as previously defined, in the offspring of the subject, from a second population of gametes (or a subpopulation thereof) which does not contain said abnormal nucleic acid sequence.

In a particular embodiment, the abnormal nucleic acid sequence is in the coding, in the non-coding or in the regulatory sequence of a nitric oxide synthase (NOS) gene (located on chromosome 7 - NCBI Reference Sequence NG_011992.1), preferably the endothelial nitric oxide synthase (*eNOS*) gene, and the multifactorial disorder is a cardiovascular disease.

Typical mutations of the *eNOS* gene are responsible for the Thr-786Cyst (T-786C) or the Glu298Asp polymorphism in the translated amino acid sequence.

Human transcript variants of the endothelial nitric oxide synthase 3 (*eNOS 3*) gene have been identified (see in particular NCBI Reference Sequences NM_000603.4, NM_001160109.1, NM_001160110.1 and NM_001160111.1).

Typically, in the context of the present invention, the abnormal *eNOS* nucleic acid sequence comprises a point mutation, in particular the T-786C polymorphism (responsible for a decreased expression of the *eNOS* gene) or the Glu298Asp polymorphism responsible for the aminoacid replacement (Glu by Asp) at position 298 of the eNOS protein.

In a particular embodiment of the present invention, the abnormal nucleic acid sequence is either in the non-coding sequence of the *eNOS* gene, and said abnormal nucleic acid sequence comprises the T-786C polymorphism, or is in the coding sequence of the *eNOS* gene, and said abnormal nucleic acid sequence comprises the Glu298Asp missense mutation.

The T-786C polymorphism in the promoter of *eNOS* bears prognostic information and is associated with changes in markers of oxidant stress in high-risk white patients referred for coronary angiography *(*The T-786C Endothelial Nitric Oxide Synthase Genotype Predicts Cardiovascular Mortality in High-Risk Patients. Gian Paolo Rossi, Giuseppe Maiolino, Mario Zanchetta, Daniele Sticchi, Luigi Pedon, Maurizio Cesari, Domenico Montemurro, Renzo De Toni, Silvia Zavattiero, Achille C. Pessina. J.American College of Cardiology, Vol 48, Issue 6, 1166-1174 (2006)).

The method preferably comprises a step of contacting the gametes of the subject with at least one discriminating substance capable of (i) altering or destroying the first population of gametes (or a subpopulation thereof), or of (ii) allowing the identification of the first or of the second population of gametes (or subpopulations thereof), thereby discriminating the first and second populations (or subpopulations) of gametes.

Preferably, the substance is a substance allowing the identification of the first or of the second population of spermatozoids (or subpopulations thereof), by altering specifically the mobility of one of said populations of spermatozoids.

A discriminating substance capable of altering the mobility of the 'second population' of spermatozoids may be selected from L-arginine, L-N⁵-(1-iminoethyl) ornithine (L-NIO), 7-nitroindazole (7-NI), N-monomethyl-D-arginine (D-NMMA), N-monomethyl-L-arginine (L-NMMA), N^{G}-nitro-*L*-arginine, N⁵-(Iminoethyl)-L-ornithine (L-NIO), diphenyleneiodonium chloride (DPI), S-methyl-1-thiocitrulline (SMTC), S-methylisothiourea hemisulphate (SMT), tyrphostin A23, tyrphostin A25, tyrphostin AG82, genistein, orthovanadate, exogenous c-Src PTK, insulin, insulin-like growth factor-1, epidermal growth factor (EGF), basic fibroblast growth factor (basic FGF), a quinazoline, a aminopyridine, a bicyclic thienooxazepine, a derivative thereof or a functional analogue thereof.

Particular derivatives of N^{G}-nitro-*L*-arginine usable in the context of the present invention may be selected from L-NAME, N-Boc-N'*-nitro*-L-*arginine,* N-gamma-nitro-L-arginine, N-alpha-T-butoxycarbonyl-N-Gnitro-L-arginine and N^{ω}-nitro-L-arginine.

In another embodiment, the present invention encompasses the population of spermatozoids recovered or produced by a method as herein described comprising a step of recovering a particular population or sub-population of the semen sample, typically the population of spermatozoids belonging to the category herein identified as 'second population' of spermatozoids or a sub-population thereof, i.e., the population of spermatozoid which does not comprise an abnormal nucleic acid sequence involved in a genetic disorder or in a multifactorial disorder.

Such a recovered population of spermatozoids may advantageously be used in the context of assisted reproductive technique or medically assisted procreation (MAP) technique, in particular for the *in vitro* fertilization (IVF) of oocytes or for the artificial insemination of a subject, typically a human subject.

In another embodiment, the present invention encompasses the population of oocytes recovered or produced by a method as herein described comprising a step of recovering a particular population or sub-population of oocytes, typically the population of oocytes belonging to the category herein identified as 'second population' of oocytes or a subpopulation thereof, i.e., the population of oocytes which does not comprise an abnormal nucleic acid sequence involved in a genetic disorder or in a multifactorial disorder. Such a recovered 'second population' may further advantageously be used in the context of assisted reproductive technique or medically assisted procreation (MAP) technique, in particular for an *in vitro* fertilization (IVF) with sperm cells.

The recovering step may be performed using a physical discriminating method and/or a discriminating substance as herein described.

In a particular embodiment, the recovering step is performed through a centrifugation step of the cells which have been previously incubated with the discriminating substance.

In a particular example, cells carrying the normal sequence of the CFTR gene can be recovered through a washing step followed by centrifugation in density gradients after incubation of the sperm with adrenaline. Cells carrying the abnormal sequence of the gene will be absent from the recovered sample as they had been destroyed by osmotic shock in the presence of adrenaline.

In a further particular example, cells carrying the normal sequence of the eNOS gene can be separated from cells carrying the abnormal sequence of the eNOS gene by centrifugation of the sperm on a density gradient. The two populations of cells will be present in different fractions of the gradient as the result of their different mobility due to their different reactions towards the inhibitor or stimulator of eNOS.

In a further embodiment, the recovering step is performed by cell sorting using either fluorescence or magnetism.

In a particular example, cells carrying the normal sequence of the CFTR gene can be recovered through binding to an anti-CFTR monoclonal antibody and exposition in a second step to a second antibody (directed against the first one) which is tagged with a fluorophore (or alternatively with a magnetic bead). Binding to the second antibody allows the recovery of those cells by cell sorting.

The present disclosure further provides kits comprising any one or more of the herein-described products (a discriminating substance, a composition of several discriminating substances, a gamete sample, in particular a spermatozoids or oocytes sample, a semen sample, a population or sub-population of gametes as herein defined, incubation media, culture media, etc.). Typically, the kit comprises at least one, preferably two products described in the present invention. Generally, the kit also comprises one or more containers filled with one or more of the substances herein disclosed. Associated with such container(s), a labelling notice may be added providing instructions for using the substances according to the present methods, specifically when considering a particular disease or disorder.

Other aspects and advantages of the invention will become apparent in the following examples, which are given for purposes of illustration and not by way of limitation.

### EXAMPLE

### Separation of the spermatozoids carrying the abnormal CFTR amino acid sequence (mutation ΔF508) responsible for the onset of cystic fibrosis, from the spermatozoïds carrying the normal CFTR protein sequence, both present in the sperm of a healthy heterozygous carrier.

### Materials and methods

### Collection of sperm and spermatozoids

Sperm was obtained by masturbation from a healthy carrier, the carrier being heterozygous for the ΔF508 mutation on the CFTR gene. Aliquots from the sperm sample were used for spermogram and spermocytogram standard analysis under the microscope. Sperm was centrifuged on a density gradient made of SupraSperm (*MediCult*). The fraction corresponding to the viable spermatozoids (standard high mobility fraction) was recovered and frozen in 600µl aliquots (50% volume of spermatozoids and 50% vol of SpermFreeze (*FertiPro*) (composition : Hepes buffer, 0,4% fetal serum Albumin Thawing of spermatozoids was made by adding 300µl of EMC culture medium (*Irvine Scientifique*) (ECM contains Dextran 10%, Bovine serum albumin 5mg/ml and Gentamicine sulfate) prewarmed at 35°C, followed by centrifugation 10min at 900g.

The pellet containing the spermatozoids was resuspended in 300µl EMC.

### Immunofluorescence

After thawing, spermatozoids were recovered by centrifugation 10 min at 900g.

The pellet, containing the spermatozoids was washed with PBS 1X (1ml) and then fixed with 1ml PBS 1X, PFA 4% (PFA: paraformaldehyde) over a microscope slide previously coated with poly-L-lysine.

The slides were incubated during 2 days in the dark to allow sedimentation and binding/fixation of the sperm cells on the slides.

Spermatozoids were then permeabilized with PBS 1X, 0,5% Triton during 1h at room temperature and then treated with PB S 1X, 5% B SA, 0,15% Glycine during 1h at room temperature.

Slides (with the spermatozoids) were washed 2 times with PBS 1X.

Spermatozoids were labelled overnight at 4°C with a 1/100 dilution in PBS 1X of a first monoclonal antibody specifically directed against the CFTR protein (mouse monoclonal anti-CFTR antibody CF3 (*Abcam*) directed against the domain of CFTR corresponding to amino acids 103-117 of SEQ ID NO: 1, referenced by Swiss-Prot under number P13569.3).

Slides were then washed 6 times, during 10 min each time, with PBS 1X.

Spermatozoids were then incubated with a 1/250 dilution of a second fluorescent antibody specifically directed against the previously mentioned first mouse antibody [goat antibody, Alexafluor488 (Invitrogen)] in PBS 1X during 1h, at room temperature, in the dark.

Slides were washed with PB S 1X 6 times, during 10 min each time, in the dark.

The slides were mounted on StarFrost microscopy slides in Vectashield containing DAPI (Vectorlabs) for labelling of cellular DNA.

Slides were observed under a Leica microscope.

### Separation of populations of spermatozoids

1x10⁶ spermatozoids were incubated for 1 h at 4°C with 100 µl of EMC (*Irvine Scientifique*) containing a 1/100 dilution of a monoclonal antibody directed against the CFTR protein (mouse monoclonal anti-CFTR antibody CF3 (*Abcam*) directed against the domain of CFTR corresponding to amino acids 103-117 of SEQ ID NO: 1). Spermatozoids were then washed with 1 ml EMC and centrifuged 5 min, at 900g. The pellet containing the spermatozoids was recovered and incubated during 30min in 100µl EMC containing a 1/100 dilution of a second fluorescent antibody specifically directed against the previously mentioned mouse antibody [goat antibody, Alexafluor488 (Invitrogen)] in PBS 1X, during 30 min, at +4°C, in the dark. Spermatozoids were then washed with 1 ml EMC and centrifuged during 5 min at 900g.The pellet was recovered in 200µl EMC on ice, in the dark.

Separation or sorting was performed using a MoFlo *(BeckmanCoulter,* buse de sortie 70µm and 15 PSI) equipment.

The different populations of spermatozoids were recovered in 200µl isotonic NaCl-EMC.

### Microscopic observation of fluorescence after cell sorting

The fractions of spermatozoids obtained after the fluorescence-based cell sorting were recovered in isotonic NaCl-EMC and centrifuged 10 min, at 900g.

The pellet containing the spermatozoids was washed with PBS 1X (1ml) and then fixed with 1ml PBS 1X, PFA 4% (PFA: paraformaldehyde) over a microscope slide previously coated with poly-L-lysine.

The slides were incubated during 2 days in the dark to allow sedimentation and binding/fixation of the sperm cells on the slides.

The slides were mounted on StarFrost microscopy slides in Vectashield containing DAPI (Vectorlabs) for labelling of cellular DNA.

Slides were observed under a Leica microscope.

### Genetic analysis

A genetic analysis (presence or absence of the ΔF508 mutation on the CFTR gene) was performed by quantitative PCR (qPCR) on genomic DNA extracted form the different populations of spermatozoids separated form the initial sperm sample.

Genomic DNA was first extracted from sperm cells by using rge commercial QIAamp DNA Mini Kit (*QIAGEN*). DTT 1M and proteinase K are added during the lysis step. The detection and quantification of the ΔF508 mutation was performed by Fast SYBR Green PCR (*Applied Biosystems*) using a Applied 7900HT thermocycler and the primers described by Ferrie et al. (1992 - see Table 3):
For the normal allele:
   Antisens sequence: GACTTCACTTCTAATGATGATTATGGGAGA3' (ΔF-Common in Ferrie et al, 1992), herein identified as SEQ ID NO: 2.

Sens sequence: GTATCTATATTCATCATAGGAAACACCACA (ΔF-j-Normal in Ferrie et al, 1992), herein identified as SEQ ID NO: 3.

For the mutant allele:
Antisens sequence: GACTTCACTTCTAATGATGATTATGGGAGA (SEQ ID NO: 2) Sens sequence: GTATCTATATTCATCATAGGAAACACCATT (ΔF-w-Mutant in Ferrie et al, 1992), herein identified as SEQ ID NO: 4.

### Observations

Sperm obtained from a healthy carrier, heterozygous for the ΔF508 mutation on the CFTR gene was centrifuged on a density gradient made of SupraSperm to obtain the viable spermatozoids (high mobility fraction). The ΔF508 mutation blocks the expression of the CFTR protein in the cell membrane.

Spermatozoids were recovered in EMC medium, fixed on microscopic slides and labelled for immuno-staining with a specific anti-CFTR monoclonal antibody. After washing, cells were labelled with a second fluorescent specific anti-anti-CFTR antibody (green fluorescence). Cells were simultaneously labelled for the presence of DNA (blue fluorescence). Figure 1A shows, as a control, and as expected to be in the sperm from a heterozygous donor, the presence of both spermatozoids expressing the CFTR membrane protein (green and blue fluorescence) and spermatozoids which do not express the CFTR membrane proteins (blue fluorescence only).

Alternatively, spermatozoids obtained from the density gradient were incubated in EMC with a specific anti-CFTR monoclonal antibody. After washing, cells were labelled with a second fluorescent specific anti-anti-CFTR antibody (green fluorescence).

Separation of spermatozoids labelled with the antibodies (i.e. expressing the CFTR on their membrane) from the non-labelled ones (i.e. not expressing the CFTR protein on their membrane) was performed using cell sorter equipment.

Figure 1B shows the populations of living spermatozoids separated by the procedure described above. Both populations comprise about 50% of the cells.

Samples from both populations were treated and labelled according to the protocol described previously corresponding to Figure 1A.

Figure 1B and 1C show that cells in one of the two populations express the CFTR protein in the plasma membrane (green and blue fluorescence) while cells of the other population of spermatozoids do not express the CFTR protein in their membranes (blue fluorescence only).

### REFERENCES

- Köhler G, Milstein C. Continuous cultures of fused cells secreting antibody of predefined specificity. Nature. 1975 Aug 7;256(5517):495-7.
- White A, Anderson DC, Daly JR Production of a highly specific monoclonal antibody progesterone. J Clin Endocrinol Metab. 1982 Jan;54(1):205-7.
- Nakamura RM., Monoclonal antibodies: methods and clinical laboratory applications. Clin Physiol Biochem. 1983;1(2-5):160-72.
- Yokoyama WM. Production of monoclonal antibodies. Curr Protoc Cell Biol. 2001 May;Chapter 16:Unit 16.1.
- Riechmann L, Clark M, Waldmann H, Winter G. Reshaping human antibodies for therapy. Nature. 1988 Mar 24;332(6162):323-7.
- Richard M. Ferrie, Martin J. Schwartz, Nancy H Robertson, Sally Vaudin, Maurice Super, Geraldine Malone and Stephen Little. "Development, Multiplexing, and Application of ARMS Tests for Common Mutations in the CFTR Gene. (Am. J. Hum. Genet. 51:251-262, 199; pp. 251-262).

## Claims

1. A method of separating gametes of a subject, which method comprises discriminating a first population of gametes containing an abnormal nucleic acid sequence involved in a genetic disease or in a multifactorial disorder in the offspring of the subject, from a second population of gametes which does not contain said abnormal nucleic acid sequence.

2. The method according to claim 1, comprising a step of contacting the gametes of the subject with at least one discriminating substance capable of (i) altering or destroying the first population of gametes, or of (ii) allowing the identification of the first or of the second population of gametes, thereby discriminating the first and second populations of gametes.

3. The method according to claim 1 or 2, wherein the method further comprises a step of recovering the second population of gametes.

4. The method according to anyone of claims 1 to 3, wherein the genetic disease is selected from a polygenic disease, a single gene disease, an autosomal dominant disease, an autosomal recessive disease, a X-linked dominant disease and a X-linked recessive disease.

5. The method according to claim 4, wherein the abnormal nucleic acid sequence is in the gene encoding CFTR (cystic fibrosis transmembrane conductance regulator) and the genetic disease is cystic fibrosis.

6. The method according to claim 5, wherein the discriminating substance is selected from epinephrine, forskolin, an anti-CFTR antibody, and a functional analogue thereof.

7. The method according to claim 4, wherein the genetic disease is a myopathy and the abnormal nucleic acid sequence is in the coding or in the regulatory sequence of a gene selected from a gene encoding a calpain, a gene encoding a dystrophin, the gene encoding Wiskott-Aldrich Syndrome Protein (WASp) and the SGCG gene encoding gamma-sarcoglycan.

8. The method according to anyone of claims 1 to 3, wherein the multifactorial disorder is selected from a cardiovascular disease, a cancer, a metabolic disease, a neurological disease, an immunological disease and a haematological disease.

9. The method according to anyone of claims 1 to 3, wherein the abnormal nucleic acid sequence is in the coding or in the regulatory sequence of a nitric oxide synthase (NOS) gene, preferably the endothelial nitric oxide synthase (eNOS) gene, and the multifactorial disorder is a cardiovascular disease.

10. The method according to claim 9, wherein the abnormal nucleic acid sequence is either in the non-coding sequence of the eNOS gene, and said abnormal nucleic acid sequence comprises the T-786C polymorphism, or is in the coding sequence of the eNOS gene, and said abnormal nucleic acid sequence comprises the Glu298Asp polymorphism.

11. The method according to claim 9 or 10, wherein the gametes are spermatozoids and the discriminating substance is a substance altering the mobility of the first or of the second population of spermatozoids.

12. The method according to claim 11, wherein the discriminating substance is a substance altering the mobility of the second population of spermatozoids and said discriminating substance is selected from L-arginine, L-N⁵-(1-iminoethyl) ornithine (L-NIO), 7-nitroindazole (7-NI), N-monomethyl-D-arginine (D-NMMA), N-monomethyl-L-arginine (L-NMMA), N^{G}-nitro-*L*-arginine, N⁵-(Iminoethyl)-L-ornithine (L-NIO), diphenyleneiodonium chloride (DPI), S-methyl-1-thiocitrulline (SMTC), S-methylisothiourea hemisulphate (SMT), a quinazoline, an aminopyridine, a bicyclic thienooxazepine, a derivative thereof and a functional analogue thereof.

13. The method according to anyone of the preceding claims, wherein the subject is a mammal, in particular a human being.

14. The population of gametes recovered by the method according to anyone of claims 3 to 13.

15. Use of at least one substance discriminating, in a sample of gametes of a subject, a first population of gametes containing an abnormal nucleic acid sequence involved in a genetic disorder or in a multifactorial disorder in the offspring of the subject, from a second population of gametes which does not contain said abnormal nucleic acid sequence, for separating gametes of a subject.
